(19) 

Europäisches
Patentamt
European
Patent Office
Office européen
des brevets

(11) **EP 4 197 508 A1**

(12) **EUROPEAN PATENT APPLICATION**

(43) Date of publication:
**21.06.2023 Bulletin 2023/25**

(21) Application number: **21214838.1**

(22) Date of filing: **15.12.2021**

(51) International Patent Classification (IPC):
**A61F 13/505** (2006.01)   **A61F 13/70** (2006.01)
**A61F 13/78** (2006.01)   **A61F 13/80** (2006.01)

(52) Cooperative Patent Classification (CPC):
**A61F 13/505; A61F 13/70; A61F 13/78;**
**A61F 13/80;** A61F 2013/5055

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Ontex BV**
**9255 Buggenhout (BE)**

• **Ontex Group NV**
**9320 Erembodegem (BE)**

(72) Inventors:
• **COBBAERT, Dries**
**1770 Liedekerke (BE)**
• **WEBER, Ainas**
**53474 Bad Neuenahr-Ahrweiler (DE)**

(74) Representative: **Saurat, Thibault**
**Ontex BV**
**Legal Department**
**Korte Keppestraat 21**
**9320 Erembodegem (BE)**

(54) **ARRAY OF DISPOSABLE INSERTS FOR ABSORBENT ARTICLES**

(57)    The present invention relates to an array of disposable inserts (25,26) for an absorbent assembly (1), the array comprising:
• a first package comprising a plurality of first inserts (25) for an absorbent assembly (1), configured to be arranged on or at least partially within an outer shell (2);
• a second package comprising a plurality of second inserts (26) for absorbent assembly (1), configured to be arranged on or at least partially within an outer shell (2);
• each first and second insert (25,26) comprising a topsheet (8), a backsheet (9) and an absorbent core (10) comprising absorbent material (23) arranged in between the topsheet (8) and backsheet (9);
the first insert (25) comprising a first absorbency as measured by method and the second insert (26) comprising a second absorbency as measured by method; wherein, the first absorbency is greater than the second absorbency. The invention also pertains to an absorbent assembly (1) and to an array of absorbent assemblies (1).

**FIG. 6**

EP 4 197 508 A1

## Description

### TECHNICAL FIELD

The present disclosure pertains to an array of disposable inserts comprising two or more disposable inserts for an absorbent assembly. Specifically, the array comprises: a first package comprising a plurality of first disposable inserts for absorbent assemblies and a second package comprising a plurality of second disposable inserts for absorbent assemblies, the first and second inserts being having different absorbency levels, different sizes and/or having different features. More generally, the present disclosure pertains to the technical field of absorbent hygiene products and relates to removable absorbent cores that can be used within an absorbent article for absorbing body fluids and exudates. The invention also relates to an absorbent assembly comprising said inserts and an outer shell as well as an array of such absorbent assemblies.

### BACKGROUND

Disposable absorbent articles, for example in the form of incontinence liners, baby diapers and sanitary napkins, are well known. The general purpose of such absorbent articles is to absorb, distribute and store various types of body exudates, while providing a high level of comfort and sense of dryness to the wearer during use of the absorbent article.

As a possibility to reduce the environmental footprint, some absorbent articles commonly called hybrid diaper comprising a re-usable part have been envisaged in an effort to decrease the amount of single-use material. For instance publication US 7,431,716 discloses a disposable diaper including a removable absorbent insert and a separate outer shell that can secure the absorbent insert to the body.

A hybrid diaper, in particular the disposable absorbent insert, is designed with an absorption capacity which is adapted to absorb the discharges that is expected to be released into the absorbent article when it is worn. However the amount of discharges varies greatly between day-time and night-time. For instance, care-givers tend to change absorbent articles more often during the day than during the night, hence is it preferable to have disposable inserts that are adapted for day-time and for night-time.

Therefore, it might be opportune for a care-giver to have both day-time and night-time disposable inserts in the same array to have better protection and comfort for the wearer depending on the time of the day.

### SUMMARY

The invention pertains to an array of disposable inserts for an absorbent assembly, the array comprising:

- a first package comprising a plurality of first inserts for an absorbent assembly, configured, or dimensioned, to be arranged, meaning to be fitted, on or at least partially, preferably entirely, within an outer shell;
- a second package comprising a plurality of second inserts for an absorbent assembly, configured, or dimensioned, to be arranged, meaning to be fitted, on or at least partially, preferably entirely, within an outer shell;
- each first and second insert comprising a topsheet, a backsheet and an absorbent core comprising absorbent material arranged in between the topsheet and backsheet;

the first insert comprising a first absorbency as measured by method mentioned herein and the second insert comprising a second absorbency as measured by method mentioned herein; wherein the first absorbency is greater than the second absorbency.

By having an array of disposable inserts that have different absorption capacities, or absorbency, meaning different performances in terms of absorption, the care-giver can provide better care by placing an insert within a re-usable outer shell that will be best adapted for the situation or the time of the day. Of course, it is also possible for a care-giver to place a night-time insert during the day if the wearer will be outside with limited access to restrooms.

According to an embodiment, the absorbent material comprises superabsorbent polymers comprising synthetic hydrogel-forming polymers (SAP) and/or superabsorbent polymers comprising natural hydrogel-forming polymers (bio-SAP).

According to an embodiment, the first inserts comprise a greater amount of absorbent material than the second inserts, preferably the first inserts comprise a greater amount of superabsorbent polymers comprising synthetic hydrogel-forming polymers (SAP) and/or superabsorbent polymers comprising natural hydrogel-forming polymers (bio-SAP).

According to an embodiment, the first inserts comprise superabsorbent polymers comprising synthetic hydrogel-forming polymers (SAP) whereas the second inserts comprise superabsorbent polymers comprising natural hydrogel-forming polymers (bio-SAP).

According to an embodiment, the first inserts comprise first superabsorbent polymer grades (SAP1) that has an Absorption Under Load (AUL) greater than 15 g/g, whereas the second inserts comprise second superabsorbent polymer grades (SAP2) that has an Absorption Under Load lesser than 15 g/g.

According to an embodiment, the first inserts comprise a first acquisition distribution layer arranged between the absorbent core and the topsheet with a first basis weight and height whereas the second inserts comprise a second acquisition distribution layer arranged between the absorbent core and the topsheet, said second acquisition distribution layer having a basis weight or a height that is lesser than the basis weight or height of the first acquisition distribution layer or whereas the second inserts comprise an absorbent core that is directly bonded to the topsheet.

According to an embodiment, the first inserts comprise an absorbent core comprising at least one channel substantially free of absorbent material whereas the second inserts comprise channels substantially free of absorbent material covering a bigger surface area.

According to an embodiment, the first inserts comprise a first channel area to core area ratio and the second inserts comprise a second channel area to core area ratio, wherein the first channel area to core area ratio is lesser than the second channel area to core area ratio, preferably at least 15% lesser, preferably at least 20% lesser, more preferably at least 30% lesser, more preferably between 40% and 70% lesser. It is preferable to have optimal balance for the core between channel area and core area, i.e. area free of channel. Enlarging the channel area might ensure a better performance in terms of acquisition speed but it results in having less space for the area free of channel and thus a smaller core area to apply the absorbent material, ultimately leading to lack of performance in terms of absorption. According to another embodiment, the first inserts do not comprise any channel substantially free of absorbent material whereas the second inserts comprise at least one channel substantially free of absorbent material.

According to an embodiment, the first inserts comprise at least one channel substantially free of absorbent material comprising a permanent bonding whereas the second inserts comprise at least one channel substantially free of absorbent material comprising a temporary bonding.

According to an embodiment, the absorbent material comprises cellulosic fibers wherein the first inserts comprise a first amount of cellulosic fibers and the second inserts comprise a second amount of cellulosic fibers, the first amount of cellulosic fibers being greater than the second amount of cellulosic fibers.

According to an embodiment, the first inserts have a first volume and the second inserts have a second volume, the first volume being greater than the second volume.

According to an embodiment, the first inserts have first additives such as skin care additives and/or the second inserts have second additives such as odor control additives, the first additives being different than the second additives.

According to an embodiment, the first inserts comprise longitudinal containment flaps and preferably transversal containment flaps, wherein the second inserts have first cuffs longitudinal containment flaps.

According to an embodiment, the first inserts comprise wetness sensors and/or the second inserts comprise wetness indicators.

The invention also pertains to an absorbent assembly comprising:

- a re-usable outer shell comprising a chassis and a fastening system configured, or dimensioned, to receive and secure an insert at least partially, preferably entirely, within a compartment;
- a first or second insert as described above configured, or dimensioned, to fit at least, preferably entirely, within said compartment. In other terms, said first or second insert has substantially the same dimensions as the compartment. Preferably, the dimensions, meaning length, width and/or height, of the inserts are lesser than the dimensions, meaning length, width and/or height, of the compartment. Although, preferably, the height of the insert is lesser than the height of the compartment.

According to an embodiment, the outer shell, in particular the fastening system, comprises a flexible cuff that can be deformed to receive inserts of different sizes within the compartment. In this embodiment, it is possible to have an insert that has dimensions, *i.e.* length, width and/or height, that are greater than the dimensions, *i.e.* length, width and/or height, of the compartment. Although, preferably, the height of the insert is lesser than the height of the compartment. By "flexible", it is meant elastic.

The invention also pertains to an array of absorbent assemblies as described above, comprising:

- a first outer shell comprising a chassis and a fastening system configured to receive and secure an insert at least partially, preferably entirely, within a compartment, the compartment being defined, or delimited, by the chassis and the fastening system;
- a second outer shell comprising a chassis and a fastening system configured to receive and secure an insert at least partially, preferably entirely, within a compartment, the compartment being defined, or delimited, by the chassis and the fastening system;

the first outer shell comprising a greater size, meaning dimensions, meaning length, width and/or height, than the second outer shell;
wherein the first or second insert are configured, or dimensioned, to fit at least partially, preferably entirely, within the compartment of the first or second outer shell.

Other objects and advantages of this invention will become apparent hereinafter.

## BRIEF DESCRIPTION OF THE FIGURES

FIG. 1 illustrates an absorbent assembly comprising a re-usable outer shell and a disposable insert;

FIG. 2 illustrates a diagrammatic top view of an absorbent assembly comprising a re-usable outer shell and a disposable insert;

FIG. 3 shows a schematic transversal cross section of an absorbent assembly comprising a re-usable outer shell and a disposable insert;

FIG. 4 and 5 illustrate diagrammatic top views of absorbent cores comprised in the disposable inserts according to embodiments herein; and

FIG. 6 shows an array of disposable inserts according to the invention.

**DETAILED DESCRIPTION**

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

"Absorbency" and "absorption" as used herein are equivalent. Absorbency is the capacity or ability to absorb liquid and absorption is the process of absorbing liquid. The two terms refer to the same capacity of the insert, or absorbent material, namely the capacity of the insert, or absorbent material, to absorb a certain amount of liquid. Said terms, "absorbency" and "absorption", both indicate the performance of an insert, or absorbent material, in terms of absorbing liquid.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Absorbent article" or "absorbent assembly" refers to devices that absorb and contain liquid, and more specifically, refers to devices that are placed against or in proximity to the body of the wearer to absorb and contain the various exudates discharged from the body. Absorbent articles, or absorbent assemblies, include but are not limited to diapers, adult incontinence briefs, training pants, diaper holders and liners, sanitary napkins and the like, as well as surgical bandages and sponges. Absorbent articles, or absorbent assemblies, preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles or absorbent assemblies comprising a re-usable cover and a disposable insert, the disposable article, or disposable insert, can include a liquid pervious topsheet, a backsheet joined to the topsheet, and an absorbent core positioned and held between the topsheet and the backsheet. The topsheet, backsheet and the absorbent medium could be made from any suitable material known to the person skilled in the art. The topsheet is generally located at or near the bodyside surface of the article, while the backsheet is generally located at or near the garment-side surface of the article. The topsheet is operatively permeable to the liquids that are intended to be held or stored by the absorbent article, or absorbent assembly, and the backsheet may or may not be substantially impervious or otherwise operatively impermeable to the intended liquids. The absorbent article, or absorbent assembly, may also include other components, such as liquid wicking layers, liquid intake layers, liquid distribution layers, transfer layers, barrier layers, wrapping layers and the like, as well as combinations thereof. Disposable absorbent articles, or absorbent assembly, and the components thereof can operate to provide a body-facing surface and a garment-facing surface.

An absorbent article or absorbent assembly, such as a hybrid diaper (infant or adult) or a hybrid pant, comprises a front waistband region, a back waistband region, an intermediate crotch region which interconnects the front and rear waistband regions. When used herein, reference to a "front" portion refers to that part of the absorbent article which is generally located on the front of a subject, such as an infant or adult, when in use. Reference to the "rear" or "back" "portion" or "section" refers to the portion of the absorbent article generally located at the rear of the subject, such as an infant or adult, when in use, and reference to the "crotch" portion refers to that portion which is generally located between the legs of subject, such as an infant or adult, when in use. The crotch region is an area where repeated fluid surge typically occurs, within the absorbent article assembly.

"Front", "rear" or "back", and "crotch" portions of the absorbent core as used herein typically refer to portions of the absorbent core that are proximal to respective portions of the absorbent article. For example, the "front" portion of the core is that which is most proximal to the front of the subject when worn, the "rear or back" portion of the core is that which is most proximal to the rear or back of the subject when worn, and the "crotch" portion of the core is the middle portion of the absorbent core between the "front" and "rear or back" portions.

The terms "configured to", "adapted to", "dimensioned to" when referring to an insert typically mean that the insert comprises features to fit or to be arranged on or at least partially, preferably entirely, within a compartment, the insert can comprise a fastening element such as glue to stick onto the outer shell or the insert can comprise given dimensions to fit within the compartment. By dimension it is meant length, width and/or height (or thickness).

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Mechanical bond" or "mechanical bonding" is an attachment between two or more elements, components, regions, or webs and may comprise heat bonds, pressure bonds, ultrasonic bonds, dynamic mechanical bonds, or any other suitable non-adhesive attachment means or combinations of these attachment means as are known in the art.

"Acquisition and distribution layer", "ADL", "Acquisition and distribution system" or "surge management portion" refers herein to a sub-layer which preferably is a nonwoven wicking layer under the topsheet of an absorbent assembly (or absorbent article), which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the topsheet and the retention portion.

The term "adhesive" as used herein is intended to refer to any suitable hot melt, water or solvent borne adhesive that can be applied to a surface of a product component of the present disclosure. Accordingly, suitable adhesives include conventional hot melt adhesives, pressure-sensitive adhesives and reactive adhesives (i.e., polyurethane). Suitable adhesives comprise a polymeric material to provide cohesive strength such as aliphatic polyolefins (such as poly (ethylene-co-propylene) copolymer); ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present disclosure can also include coform, which is produced by combining air-entrained dry, dispersed cellulosic fibers with meltblown synthetic polymer fibers while the polymer fibers are still tacky.

As used therein, the term "associated" encompasses configurations in which topsheet is directly joined to backsheet by affixing topsheet directly to backsheet, and configurations wherein topsheet is joined to backsheet by affixing topsheet to intermediate members which in turn are affixed to backsheet. Topsheet and backsheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet to backsheet. It should be readily appreciated that the above-described attachment means may also be employed to interconnect and assemble together the various other component parts of the article described herein.

The term "backsheet" or "back sheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing there through. Examples of breathable backsheet materials are porous polymeric films, nonwoven laminates of spunbond and meltblown layers and laminates of porous polymeric films and nonwoven materials.

The term "blend" means a mixture of two or more polymers while the term "alloy" means a sub-class of blends wherein the components are immiscible but have been compatibilized.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to films having a water vapor transmission rate (WVTR) of at least 300 grams/m$^2$ - 24 hours.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods such as powder bonding; pattern bonding, air-through bonding, chemical bonding and consolidation by mechanical methods such as needling and hydroentanglement. Carded thermobonded nonwoven thus refers to a carded nonwoven wherein the bonding is achieved by use of heat.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Coform" as used herein is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent particles.

The term "density" or "concentration" when referring to the absorbent material, in particular the SAP, of a layer, refers to the amount of the absorbent material divided by the surface area of the layer over which the absorbent material is spread out.

The term "disposable" is used herein to describe absorbent articles, or absorbent assemblies, that generally are not intended to be laundered or otherwise restored or reused as an absorbent article (i.e., they are intended to be discarded after a single use and, preferably, to be recycled, composted or otherwise disposed of in an environmentally compatible manner) as opposed to re-usable or washable.

As used herein, the terms "elastic", "elastomer", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length.

The term "elasticized" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent), as represented by the following: "Extension" means the change in length of a material due to stretching (expressed in units of length).

The term "fabrics" is used to refer to all of the woven, knitted and nonwoven fibrous webs.

As used herein, the term "garment" means any type of apparel which may be worn. This includes diapers, training pants, incontinence products, surgical gowns, industrial workwear and coveralls, undergarments, pants, shirts, jackets and the like.

Many of the known superabsorbent polymer particles exhibit gel-blocking. "Gel-blocking" occurs when superabsorbent polymer particles are wetted and the particles swell so as to inhibit fluid transmission to other regions of the absorbent structure. Wetting of these other regions of the absorbent member therefore takes place via a very slow diffusion process. In practical terms, this means acquisition of fluids by the absorbent structure is much slower than the rate at which fluids are discharged, especially in gush situations. Leakage from the absorbent article can take place well before the particles of SAP in the absorbent member are even close to being fully saturated or before the fluid can diffuse or wick past the "blocking" particles into the rest of the absorbent member. Gel-blocking can be a particularly acute problem if the superabsorbent polymer particles do not have adequate gel strength and deform or spread under stress once the particles swell with absorbed fluid.

"Hydroentanglement process" refers to the manufacturing of nonwoven webs. The process involves directing a series of water jets towards a fibrous web which is supported on a moving porous belt. The water jets pass downwards through the mass of fibers and on making contact with the surface of the belt, the jets rebound, and break up: the energy released causes entanglement of the mass of fibers.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, or the like.

A layer can be liquid and air permeable, permeable to air but impermeable to liquids, impermeable both to air and liquid, or the like. When used in the singular, it can have the dual meaning of a single element or a plurality of elements.

The crotch portion of the absorbent article such as incontinence pants or pads preferably comprises opposite longitudinal side portions which comprise a pair of elasticized, longitudinally extending "leg cuffs". The leg cuffs are generally adapted to fit about the legs of a wearer when in use and serve as a mechanical barrier to the lateral flow of body exudates. Leg cuffs are elasticized by leg elastics. The incontinence pant further can comprise a front waist elastic and a rear waist elastic. Materials suitable for use in forming leg elastics are known to those skilled in the art. Exemplary of such materials are strands or ribbons of a polymeric, elastomeric material which are adhered to the incontinence pant at the leg cuff while in a stretched position, or which are attached to the incontinence pant while the incontinence pant is pleated, such that elastic constrictive forces are imparted to the leg cuff. Examples of suitable elastomer materials that can be used include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

"Liquid" means a nongaseous substance and/or material that flows and can assume the interior shape of a container into which it is poured or placed.

"Longitudinal" is a direction running parallel to the maximum linear dimension of the article.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "natural" when referring to a hydrogel-forming polymeric absorbent material typically means that said material is existing in or derived from nature or in other words that is not made or caused by humankind. The terms "organic" is equivalent to "natural" and typically means that said hydrogel-forming polymeric absorbent material is being produced or involving production without the use of synthetic chemicals. We can also say that the hydrogel-forming polymeric absorbent material is derived from biomass or bio-sourced.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp", "cellulosic fluff" or "fluff" refers to a material made up of cellulose fibers. The fibers can be either natural or synthetic, or a combination thereof. The material is typically lightweight and has absorbent properties.

The "retention portion" or "liquid absorption layer" is part of the absorbent medium. This portion may comprise a matrix of hydrophilic fibers, such as a web of cellulosic fluff, mixed with particles of high absorbency material. In particular arrangements, the retention portion may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-stepwise gradient through a substantial portion of the thickness of the absorbent structure, with lower concentrations toward the bodyside of the absorbent structure and relatively higher concentrations toward the outer side of the absorbent structure. The superabsorbent particles may also be arranged in a generally discrete layer within the matrix of hydrophilic fibers. In addition, two or more different types of superabsorbent may be selectively positioned at different locations within or along the fiber matrix.

As used herein the term "sheet" or "sheet material" refers to woven materials, nonwoven webs, polymeric films, polymeric scrim-like materials, and polymeric foam sheeting.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 $\mu$m or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be preformed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

As used herein "channels" are fluid distribution means within the absorbent core adapted to favor exudate flow there along and are typically intended to exclude embossing patterns or ducts formed by compression from the meaning thereof and rather include structures that are substantially free of absorbent material instead of comprising compacted absorbent material. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1mm, preferably from 5mm to 50mm, more preferably from 6mm to 40mm, more preferably from 8mm to 30mm, even more preferably from greater than 8mm to less than 25mm.

By "substantially", it is meant at least the majority of the structure referred to. For example, with reference to a channel following "a substantially continuous path from any point of said channel to any other point of the same channel", this means that the majority of the channel is interconnected and generally wherein a direct and continuous path can be traced by starting from one point of the channel towards another point of the channel.

Use of the term "substrate" includes, but is not limited to, woven or nonwoven webs, porous films, ink permeable films, paper, composite structures, or the like.

The terms "superabsorbent" or "high absorbency" refer to materials that are capable of absorbing at least 10 times their own weight in liquid. Superabsorbent materials suitable for use in the present disclosure are known to those skilled in the art, and may be in any operative form, such as particulate form, fibers and mixtures thereof. Generally stated, the "superabsorbent material" can be a water-swellable, organic or inorganic, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The natural, or organic, hydrogel-forming polymeric absorbent material may be formed from natural, or organic, hydrogel-forming polymeric material, which may include natural material such as agar, pectin and the natural gums, such as alginates, xanthan gum, locust bean gum, guar gum and the like; modified natural materials such as carboxymethyl cellulose, carboxyethyl cellulose, methyl cellulose and hydroxypropyl cellulose. Alternatively, the hydrogel-forming polymeric absorbent material may be formed from synthetic hydrogel-forming polymeric material. Synthetic hydrogel-forming polymeric material includes, for example, alkali metal and ammonium salts of polyacrylic acid and polymethacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, maleic anhydride copolymers with vinyl ethers and alpha-olefins, poly(vinyl pyrrolidone), poly(vinylmorpholinone), poly(vinyl alcohol), and the like, and mixtures and copolymers thereof. Other suitable synthetic hydrogel-forming polymers include hydrolyzed acrylonitrile grafted starch, acrylic acid grafted starch, and isobutylene maleic anhydride copolymers and mixtures thereof. The hydrogel-forming polymers may be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, irradiation, crystalline domains, covalent bonds, ionic complexes and associations, hydrophilic associations, such as hydrogen bonding, and hydrophobic associations or Van der Waals forces. Mixtures of natural and wholly or partially synthetic absorbent polymers can also be used. Synthetic hydrogel-forming materials typically are xerogels which form hydrogels when wetted. The term "hydrogel", however, has commonly been used to also refer to both the wetted and unwetted forms of the material. The superabsorbent material may be in any of a wide variety of geometric forms. As a general rule, it is preferred that the superabsorbent material be in the form of discrete particles. However, the superabsorbent material may also be in the form of fibers, flakes, rods, spheres, needles, spiral or semi-spiral, cubic, rod-like, polyhedral, or the like. Conglomerates of particles of superabsorbent material may also be used. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibers. Alternatively, the absorbent core may comprise a laminate of fibrous webs and high-absorbency material or other suitable means of maintaining a high-absorbency material in a localized area.

"Superabsorbent polymer particles" or "SAPs" refer to water-swellable, water-insoluble organic or inorganic materials capable, under the most favourable conditions, of absorbing at least about 10 times their weight, or at least about 15 times their weight, or at least about 25 times their weight in an aqueous solution containing 0.9 weight percent sodium chloride. In absorbent articles, such as diapers, incontinent pants or pads, etc., the particle size is typically ranging between 100 to 800 $\mu$m, preferably between 300 to 600 $\mu$m, more preferably between 400 to 500 $\mu$m.

As used herein, the term "thermoplastic" is meant to describe a material that softens when exposed to heat and which substantially returns to its original condition when cooled to room temperature.

The term "topsheet" or "top sheet" refers to a liquid permeable material sheet forming the inner cover of the absorbent article and which in use is placed in direct contact with the skin of the wearer. The topsheet is typically employed to help isolate the wearer's skin from liquids held in the absorbent structure. The topsheet can comprise a nonwoven material, e.g. spunbond, meltblown, carded, hydroentangled, wetlaid etc. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. Further examples of topsheet materials are porous foams, apertured plastic films, laminates of nonwoven materials and apertured plastic films etc. The materials suited as topsheet materials should be soft and non-irritating to the skin and be readily penetrated by body fluid, e.g. urine or menstrual fluid. The inner coversheet may further be different in different parts of the absorbent article. The topsheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction.

"Ultrasonic welding or bonding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

"Wet state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighboring particles.

By the term "concentration" of (e.g. super absorbent polymer) as used herein, is meant the amount of the referred material divided by the surface area of the layer referred to (typically said area being in the plane of the length and width of the core) over or within which the referred material is contained. This may be determined by standard weighing and dimensional measuring methods known in the art and can be expressed in g/mm$^2$.

By the term "substantially matches the shape of the channel(s)" as used herein, is meant that the feature referred to has an overlapping shape that is visually the same as the channel(s).

By the terms "longitudinally-, transversally-, diagonally--extending" as used herein, is meant a general orientation substantially parallel respectively to the longitudinal axis, to the transversal axis, and to any axis between them. This covers deviations from the axis used as reference of between 0° and 20°. This may cover straight lines but also curved lines. In the latter case, it is the main general orientation of the curve which is meant to be described.

By the term "center line" or "centre line" as used herein, is meant an axis dividing the element to which it refers, in two portions of equal length on either side of this axis.

The term "symmetrical" refers to an element having, involving, or exhibiting symmetry, *i.e.* correspondence in size, shape, and relative position of parts on opposite sides of a dividing line or median plane or about a center or axis. We can also say said element being made up of exactly similar parts facing each other or around an axis. Naturally, asymmetrical refers to the opposite, meaning an element having parts that fail to correspond to one another in shape, size, or arrangement; *i.e.* lacking symmetry.

Embodiments of the articles and processes according to the disclosure will now be described. It is understood that technical features described in one or more embodiments maybe combined with one or more other embodiments without departing from the intention of the disclosure and without generalization therefrom.

The present invention provides an array of absorbent disposable inserts.

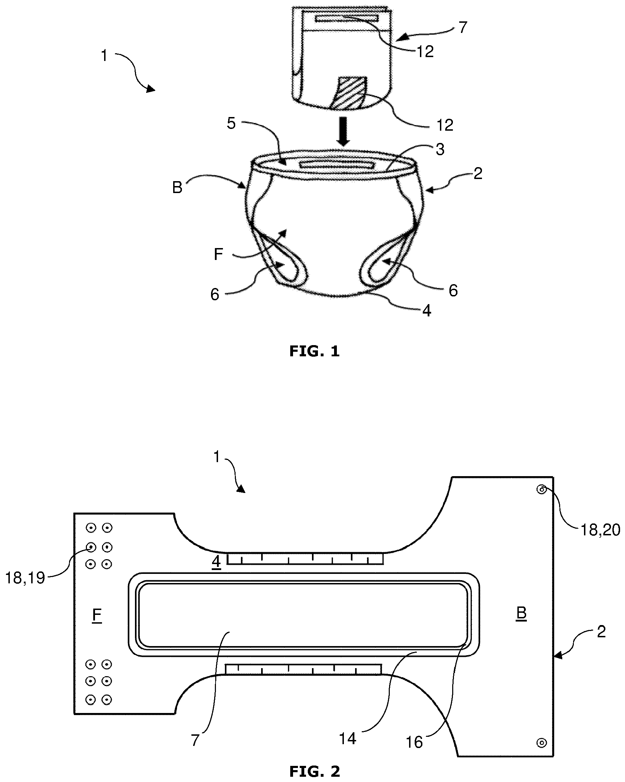

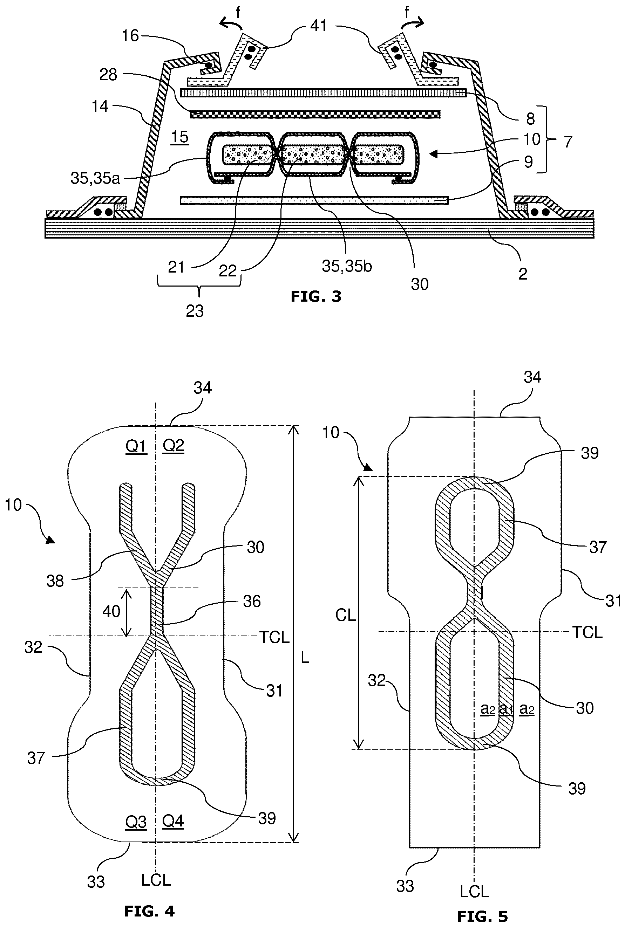

As exemplified in FIG. 1, the disclosure relates to an absorbent assembly 1 comprising a re-usable outer shell 2, or outer cover, and an absorbent disposable insert 7, the absorbent assembly 1 can also be called a hybrid diaper. The washable outer shell 2 comprises a chassis with a front F and back B waist region 3 having uppermost and lowermost edges, and a crotch region 4 interposed between the front F and back B waist region 3. The uppermost edges of the waist region 3 form a waist opening 5 and the lowermost edges of the waist region 3 together with lateral extremities of the crotch region 4 form two oppositely disposed leg openings 6. The disposable absorbent insert 7 comprises a liquid permeable topsheet 8, a liquid impermeable backsheet 9, and an absorbent core 10 interposed between said topsheet 8 and said backsheet 9 (Fig. 3). The topsheet 8 and backsheet 9 are associated, or joined, in order to contain, or enclose, the absorbent core 10 between said topsheet 8 and backsheet 9

The absorbent assembly 1 comprises fastening means to secure the insert 7 on or within the outer shell 2. For instance, in an embodiment the garment-facing surface of the insert 7, *i.e.* the outer side of the backsheet 9, comprises one or more fastening surfaces 12 arranged to releasably engage with the body-facing surface of the outer shell 2, the one or more fastening surfaces 12 comprising for example a strip of adhesive, preferably non-structural adhesive, so that the insert 7 can be placed and secured on the outer shell 2 and removed easily from said shell 2 once the absorbent core 10 is full. In an embodiment, the garment-facing surface of the absorbent insert 7 comprises one or more fastening surfaces 12 arranged to releasably engage with one or more fastening surfaces 12 of said outer shell 2, the one or more fastening surfaces 12 on the insert 7 comprising for example a strip of hooks whereas the fastening surfaces 12 on the outer shell 2 comprising a strip of loops or vice-versa. Preferably, the plurality of fastening surfaces are in the form of strips each being separated along the length of said absorbent insert 7, more preferably being in the form of at least three strips 12 arranged proximal to the ends of said insert 7 and at a position therebetween. In an embodiment, the fastening system comprises snap socket with the cap being arranged on the insert 7 and the socket being arranged on the outer shell 2, or vice versa.

In another embodiment of the fastening means, the outer shell 2 comprises a compartment 15, or pocket, configured, or dimensioned, to receive the insert 7, the outer shell 2 comprises flexible and resilient means that are arranged to maintain the insert 7 within the compartment 15. For instance, according to the embodiment illustrated in FIG. 2 and FIG. 3, the outer shell 2 comprises a fastening cuff 14 extending in the longitudinal and transversal directions of the outer shell 2, defining a rectangular perimeter, the fastening cuff 14 defining a compartment 15, or pocket, *i.e.* an inner space, where the insert 7 can be placed. The fastening cuff 14 extends upward, or outward, in the body-facing direction and comprises an elastic mean 16, such as an elastic ring or elastic string or elastic strap, at the outward extremities to maintain the insert 7 within the compartment 15 defined by the outer shell 2, namely the chassis, fastening cuff 14 and the elastic mean 16. In other terms, the fastening cuff 14 comprises a proximal end that is attached, or joined, to the outer shell 2 and an unattached distal end that comprises an elastic mean 16. In other terms, the chassis of the outer shell 2, the fastening cuff 14 and the elastic mean 16 define a prism, or rectangular cube, with a bottom wall (chassis) and side walls (fastening cuff 14) and an open top end opposite to the bottom wall, the inner void or empty inner space defined by said walls being the compartment 15 where to position the insert 7, the elastic mean 16 further securing said insert within the compartment 15. The fastening cuff 14 and elastic mean 16 are arranged to press against the side walls and/or upper wall (topsheet) of the insert 7. For example, the fastening cuff 14 presses against the side walls of the insert 7 and the elastic mean 16 presses against the topsheet 9 of the insert 7 meaning that the height of the insert is lesser than the height of the compartment 15. Alternatively, both the fastening cuff 14 and the elastic mean 16 press against the side walls of the insert 7, the elastic strength of the elastic mean 16 maintaining the insert 7 within the compartment 15, in this case the height of the insert 7 is greater than the height of the compartment 15. Alternatively, only the elastic mean 16 presses against the topsheet 9 of the insert 7 meaning that the length and width of the insert 7 is lesser than the length and width of the compartment 15.

The outer shell 2 comprises re-fastenable members 18 arranged on the back B waist region 3 and/or on the front F waist region 3 to fasten the back B waist region 3 to the front F waist region 3. As exemplified in FIG. 2, the outer shell 2 comprises mechanical fasteners 18 such as snap fasteners, here the front F waist region 3 comprises a plurality of sockets 19 and the back B waist region 3 comprises two caps 20 arranged at each transversal end of the outer shell 2. The cap 20 can fit, *i.e.* snap-fit, onto any of the socket 19 so that the hybrid diaper 1 can be properly fitted onto the wearer.

Alternatively, the crotch region 4 may comprise one or more re-fastenable members such that said crotch region 4 is unitary when said re-fastenable members are fastened and that said crotch region is separated into two crotch halves when said re-fastenable members are unfastened to form a crotch-release opening, and in that the disposable absorbent insert 7 may be single-handedly removed from said elastic outer cover 2 from said crotch-release opening.

The insert 7, comprises a liquid pervious topsheet 8, a liquid impervious backsheet 9 and an absorbent core 10 arranged in between said topsheet 8 and backsheet 9, the topsheet 8 and backsheet 9 being associated to one another to envelop the absorbent core 10. The absorbent core 10 is the absorbent structure comprising absorbent material 23 disposed between the topsheet 8 and the backsheet 9 of the absorbent assembly 1 in at least the crotch region 4 of the absorbent assembly 1 and is capable of absorbing and retaining liquid body exudates. The size and the absorbent capacity of the absorbent medium should be compatible with the size of the intended wearer and the liquid loading imparted by the intended use of the absorbent assembly. Further, the size and the absorbent capacity of the absorbent core 10 can be varied to accommodate wearers ranging from infants through adults. It may be manufactured in a wide variety of shapes as illustrated in FIG. 4 or FIG. 5, for example and not limited to a rectangular shape, a trapezoidal shape, a T-shape, an I-shape or an hourglass shape and from a wide variety of materials. Examples of commonly occurring absorbent materials 23 are cellulosic fluff pulp, tissue layers, highly absorbent polymers (so called superabsorbent polymer particles (SAP)), absorbent foam materials, absorbent nonwoven materials or the like. It is common to combine cellulosic fluff pulp 21 with superabsorbent polymers 22 as an absorbent material 23 as illustrated in FIG. 3.

The object of the invention is to offer the customer an absorbent assembly 1 comprising an array of inserts that are best adapted for night or day. In other terms, the invention pertains to an array of disposable inserts for absorbent assemblies, or hybrid diapers, the array comprising a first package comprising a plurality of first inserts 25 for absorbent assemblies, configured to be arranged within an outer shell 2, the first inserts 25 are for example night-time inserts. The array further comprises a second package comprising a plurality of second inserts 26 for absorbent assemblies configured to be arranged within an outer shell 2, the second inserts 26 are for example the day-time inserts. Each first 25 and second 26 inserts comprises a liquid pervious topsheet 8, a liquid impervious backsheet 9 and an absorbent core 10 comprising absorbent material 23 arranged in between the topsheet 8 and backsheet 9. According to the invention, the first inserts 25 have a greater absorbency, greater dimensions and/or additional features compared to the second inserts 26.

The object of the invention is not limited to infant diapers or incontinence adult diapers. Naturally, adult diapers are expected to have a higher absorption than baby diapers. For instance in an array of inserts for infant diapers, the first insert 25 comprise a first absorbency as measured by Method ISO 17190-6 and the second insert 26 comprising a second absorbency as measured by Method ISO 17190-6, wherein, the first absorbency is greater than the second absorbency. Preferably, the absorbency of the first inserts 25 is at least 1.5 greater than the absorbency of the second inserts 26. The absorption of the first inserts 25 is from about 250 g to about 600 g, preferably from 300 g to 550 g, more preferably from 350 g to 500 g, as measured by the Method ISO 17190-6 whereas the absorption of the second inserts 26 is from about 150 g to about 350 g, preferably from 175 g to 300 g, more prefably from 200 g to 250 g, as measured by the Method ISO 17190-6. For instance, in an array of inserts for adult diapers, the first insert 25 comprise a first absorbency as measured by Method ISO 11948-1 and the second insert 26 comprising a second absorbency as measured by Method ISO 11948-1, wherein, the first absorbency is greater than the second absorbency. Preferably, the absorbency of the first inserts 25 is at least 1.5 greater than the absorbency of the second inserts 26. The absorption of the first inserts 25 is from about 1200 g to about 2000 g as measured by the Method ISO 11948-1 whereas the absorption of the second inserts 26 is from about 800 g to about 1200 g as measured by the Method ISO 11948-1. Method ISO 17190-6:2001 and Method ISO 11948-1:1996 are described later on. It is possible to use either method to measure the absorbency of an insert, preferably Method ISO 17190-6 is used to measure the absorbency of an insert for infant diaper and Method ISO 11948-1 is used to measure the absorbency of an adult diaper.

As we have seen the discharges varies greatly between day-time and night-time and a care-giver changes absorbent articles for a wearer more often during the day than during the night. Hence it is preferable that the night-time inserts 25 present a greater absorbency level than day-time inserts 26.

A first embodiment for improving absorbency is to increase the amount of absorbent material 23 in the first inserts 25. Hence the first inserts 25 comprise a greater amount of SAP than the second inserts 26. It is also possible that the first inserts 25 comprise a greater amount of fluff than the second inserts. Of course, the first inserts 25 can comprise a greater amount of SAP and fluff than the second inserts 26.

According to the invention, the absorbent core 10 comprises absorbent material 23 selected from the group consisting of cellulose fibers 21, superabsorbent polymers 22 and combinations thereof. The absorbent material 23 may in particular comprise cellulosic fluff and/or fibrous web typically comprising airlaid fibers of the cellulosic kind. The superabsorbent polymers 22 may typically be in the form of a plurality of discrete particles that may be distributed within the absorbent material 23 or directly agglomerated in one or more pockets between at least two nonwoven webs. The absorbent core 10 may include cellulose fibers 21 and superabsorbent polymers 22 in a proportion of 5-55 Wt% cellulose fibers and 45-95 Wt% SAP. For example, in an array according to an embodiment, the first inserts 25 comprise about 75 to about 90 Wt% SAP and about 10 to about 25 Wt% cellulose fibers whereas the second inserts 26 comprise about 40 to about 70 Wt% SAP and about 30 to about 60 Wt% cellulose fibers.

Another embodiment to improve absorbency, is to alter the nature of the Superabsorbent polymers (SAP). For example, since the day-time inserts 26 are changed more often, the SAP doesn't need to be as efficient as the SAP used in the night time inserts 25. Hence, the second insert 26 can comprise bio-SAP whereas the first insert 25 comprise synthetic-SAP (see hereunder for definitions).

Superabsorbent polymers, superabsorbent polymers particles, or SAPs refer to water-swellable, water-insoluble organic or inorganic materials capable of absorbing at least about 10 times their weight, or at least about 15 times their weight, or at least about 25 times their weight in an aqueous solution containing 0.9 weight percent sodium chloride. Traditional, conventional or classic SAP, meaning synthetic-SAP, are made out of synthetic hydrogel-forming polymeric absorbent material, meaning synthetic hydrogel-forming polymers which include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable synthetic hydrogel-forming polymers include hydrolyzed acrylonitrile grafted starch, acrylic acid grafted starch, and isobutylene maleic anhydride copolymers and mixtures thereof. Bio-SAP are made out of organic or natural hydrogel-forming polymeric absorbent material, meaning organic or natural hydrogel-forming polymers which include organic or natural material such as agar, pectin, and guar gum; modified organic or natural materials such as carboxymethyl cellulose, carboxyethyl cellulose, and hydroxypropyl cellulose. Both organic or synthetic hydrogel-forming polymers may be lightly crosslinked to render the material substantially water insoluble. Synthetic-SAP tends to have greater absorbency performances than Bio-SAP.

Absorbent cores 10 comprised in the array of inserts 25, 26 of the present disclosure comprise specific SAP grades and/or types.

For instance, the first inserts 25 comprise first superabsorbent polymer grades (SAP1) that has an AUL (Absorption Under Load) greater than 15 g/g, preferably from 18 g/g to 55 g/g, even more preferably from 20 g/g to 50 g/g, even more preferably from 21 g/g to 45 g/g, even more preferably from 22 g/g to 35 g/g. The first superabsorbent polymer grades (SAP1) typically have an absorption speed, according to the test method herein, of more than 50 seconds, preferably more than 55 seconds, more preferably from 60 seconds to 150 seconds, even more preferably from 70 seconds to 100 seconds. Advantageously this allows reduced rewet (indication of dryness on the skin-facing surface) because of the high AUL on the upper surface that "releases" less liquid under load/pressure e.g. weight of the baby, with compromise on absorption speed and speed of liquid uptake. The second inserts 26 comprise second superabsorbent polymer grades (SAP2) herein that has an AUL of less than 15 g/g, preferably from 5 g/g to 15 g/g, even more preferably from 8 g/g to 12 g/g. The second superabsorbent polymer grades (SAP2) typically has an absorption speed, according to the test method herein, of less than 45 seconds, preferably from 5 seconds to 40 seconds, even more preferably from 15 seconds to 35 seconds. Advantageously this allows for extremely fast liquid uptake though with some compromise to rewet performance and uptake under load.

Of course, both inserts 25,26 can comprise synthetic-SAP or bio-SAP, however bio-SAP tends to display lower absorbency or AUL performance, hence bio-SAP is more adapted for the second inserts 26. However, in a sustainable approach, it is also possible to have bio-SAP in both first inserts 25 and second inserts 25. In other terms, both first and second inserts 25,26 comprise bio-SAP, the amount of bio-SAP being greater in the first inserts 25 than in the second inserts 26.

As seen above, according to an embodiment, the first inserts 25 and second inserts 26 both comprise SAP with different grades. Specifically, the first inserts 25 comprise first superabsorbent polymer grades (SAP1) that has an AUL (Absorption Under Load) greater than 15 g/g, whereas the second inserts 26 comprise second superabsorbent polymer grades (SAP2) that has an AUL (Absorption Under Load) lesser than 15 g/g.

Another embodiment for improving absorbency and comfort, is to ensure that the fluid distribution is optimal so that the entire surface and volume of the absorbent core is utilized to absorb the incoming liquid.

According to an embodiment, the first inserts 25 comprise a first Acquisition Distribution Layer (ADL) 28 whereas the second inserts 26 comprise a second thinner or with a lesser basis weight acquisition distribution layer 28 or even no acquisition distribution layer at all. The acquisition distribution layer 28 may be positioned at a body-facing side of the absorbent core 10, between the topsheet 8 and the absorbent core 10 of the insert 7, and more preferably in close proximity or even in good contact (most preferably in direct contact) with the body-facing side of the absorbent core 10. The use of an ADL leads to an extremely good distribution of fluids from a discharge area to the entire absorbent core 10 whilst attaining excellent perceived dryness performance.

The acquisition distribution layer 28 may be a single layer of spunbond and/or carded (e.g. carded thermobonded) nonwoven. Alternatively, the acquisition distribution layer 28 may be multi-layered and comprise at least one spunbond layer and at least one meltblown layer typically the layers being nonwoven layers. Preferably, the acquisition distribution layer 28 is a nonwoven selected from the group consisting of: SM, SMS, SMMS, and combinations thereof.

In an embodiment, the acquisition distribution layer 28 comprises a plurality of layers, wherein at least one of said layers, preferably each of said layers, consists of spunbond, meltblown and/or carded (e.g. thermocarded) nonwoven and wherein at least the layer most distal from the body-facing side of the absorbent core consists of spunbond and/or carded nonwoven, preferably wherein both the layer most distal and the layer most proximal to the body-facing (also referred to herein as "skin-facing") side of the absorbent core consists of spunbond and/or carded nonwoven.

In an embodiment, the acquisition distribution layer 28 for use herein comprises synthetic fibers which are comprised at a level of greater than 80%wt by weight of said acquisition distribution layer. The acquisition distribution layer may have a basis weight of from 5 to 50 $g/m^2$, 10 to 50 $g/m^2$, preferably from 15 to 40 $g/m^2$, more preferably from 18 to 35 $g/m^2$, even more preferably from 20 to 30 $g/m^2$, most preferably from 21 to 25 $g/m^2$. For example, the first inserts 25 can comprise an acquisition distribution layer with a basis weight from 20 to 30 $g/m^2$ whereas the second inserts comprise an acquisition distribution layer having a basis weight from 15 to 25 $g/m^2$, the basis weight of the ADL in the first inserts 25 being greater than the basis weight of the second inserts 26.

Of course, it is possible to combine several embodiments, for example the array of disposable inserts may comprise first inserts 25 having a greater amount of absorbent material 23 and an ADL 28 with a greater basis weight than the second inserts 26. Also, the array of disposable inserts may comprise first inserts 25 comprising synthetic-SAP and an ADL 28 with a greater basis weight than the second inserts 26 comprising bio-SAP.

Distribution of fluids can be improved by other means such arranging channels substantially free of absorbent material in the absorbent core. According to an embodiment the first inserts 25 comprise an absorbent core 10 comprising at least one channel 30 that is substantially free of absorbent material whereas the second inserts 26 comprise an absorbent core 10 comprising at least one channel 30 substantially free of absorbent material that covers a greater surface area. The one or more channels 30 of the first and second inserts 25,26 are arranged within the inner periphery of the absorbent core 10, i.e. the inner, or interior, space defined by the periphery of the absorbent core 10, such that each of the channel(s) 30 is bounded, or surrounded, by absorbent material 23. Additionally, the length of the channels 30 may be from 10% to 95% of the length of the absorbent core 10 so that again the one or more channels 30 are bounded by absorbent material 23.

As illustrated in FIG. 4 and FIG. 5, the absorbent core 10 in the first and second inserts 25,26 has a first and second longitudinal edge 31,32 and a front 33 and back 34 transverse edges. It has a longitudinal center line LCL dividing the absorbent core 10 in a first longitudinal portion and a second longitudinal portion on either side of the longitudinal center line LCL. It also has a transverse center line TCL dividing the absorbent core 10 in a front portion and a back portion on either side of the transverse center line TCL. The absorbent core 10 comprises absorbent material 23 selected from the group consisting of cellulose fibers, superabsorbent polymers and combinations thereof, and the absorbent material is contained within at least one core wrap 35 substrate enclosing said absorbent material 23 (FIG. 3). For example, the core wrap 35 can comprise two core wrap sheets or one sheet that is folded over itself. The top layer 35a of said core wrap 35 is adhered, or bonded, to a bottom layer 35b of said core wrap 35 to form the one or more channels 30 substantially free of said absorbent material 23. The core wrap layers are preferably nonwoven webs.

Advantageously, at least one of the top and bottom core wrap layers, or sheets, is an elastic nonwoven. At least one channel 30 follows a substantially continuous path from any point of said channel 30 to any other point of the same channel as illustrated in FIG. 4 and FIG. 5.

The absorbent core 10 of the first inserts 25 comprises a first channel area to core area ratio and the absorbent core 10 of the second inserts 26 comprises a second channel area to core area ratio. The first channel area to core area ratio is lesser than the second channel area to core area ratio, preferably at least 15% greater, preferably at least 20% greater, more preferably at least 30% greater, more preferably between 40% and 70% greater. The channel area $a_1$ (see FIG. 4) is the area defined by the channel(s) in the absorbent core 1,1' and the core area $a_2$ (see FIG. 5) is the area defined by the portion of the absorbent core that is free of channel(s). In other terms, the channel area $a_1$ is the area of the absorbent core 1,1' that is substantially free of absorbent material whereas the core area $a_2$ is the area of the absorbent core 1,1' comprising absorbent material. The first channel area to core area ratio ($a_1/a_2$) is preferably comprised between about 10 % to about 20 %, i.e. the first ratio value is comprised between about 0.1 to about 0.2, whereas the second channel area to core area ratio ($a_1/a_2$) is preferably comprised between about 5 % to about 15 % (0.05 to 0.15). For example in the array of inserts, the absorbent core 10 in the first inserts 25 comprises a first ratio $a_1/a_2$ of 11 % whereas the absorbent core 10 in the second inserts 26 comprises a second ratio $a_1/a_2$ of 17%, the first channel area to core area ratio is about 65% lesser than the second channel area to core area ratio. Again, enlarging the channel area might ensure a better performance in terms of acquisition speed but it results in having less space for the area free of channel and thus a smaller core area to apply the absorbent material, ultimately leading to lack of performance in terms of absorbency.

The at least one channel 30 preferably follows a substantially continuous path from any point of said channel to any other point of the same channel. The absorbent core 10 has a transverse center line TCL dividing the absorbent core 10 in a front portion and a back portion on either side of the transverse center line TCL and a longitudinal center line LCL dividing the absorbent core 10 in a first longitudinal portion and a second longitudinal portion on either side of the longitudinal center line LCL, thereby forming four quadrants Q1, Q2, Q3, Q4. Said channel 30 preferably comprises at least:

- one longitudinally extending central section 36 coincident with the longitudinal center line LCL,
- one longitudinally extending lateral section 37 in each of the four quadrants Q1,Q2,Q3,Q4,
- four diagonally extending diagonal sections 38, diverging from the central section end points towards the lateral sections in each quadrant and connecting said central 36 and lateral sections 37, and

- at least one transversally extending closing section 39 in one of the front and/or back portions, joining each of the two lateral sections 37 end points closest to the absorbent core respective front or back transverse edge 33,34.

In an embodiment, as for example illustrated in FIG. 4, the channel 30 comprises one transversally extending closing section 39 in the front portion, joining each of the two lateral sections 37 end points closest to the absorbent core respective front transverse edge 33. In an embodiment, as for example illustrated in FIG. 5, the channel 30 comprises two transversally extending closing sections 39, one in the front portion, the other in the back portion, each joining each of the two lateral sections 37 end points closest to the absorbent core at the front transverse edge 33 and at the back transverse edge 34 respectively. This may allow distribution of fluid beyond the second closing section itself, closer to the transverse edge of the core. According to an embodiment, the first inserts 25 comprise an absorbent core 10 with two transversally extending closing sections 39 whereas the second inserts 26 comprise an absorbent core 10 with one transversally extending closing sections 39. Alternatively, according to an embodiment, the first inserts 25 comprise an absorbent core 10 with one transversally extending closing sections 39 whereas the second inserts 26 comprise an absorbent core 10 with two transversally extending closing sections 39.

According to an embodiment, when comprising at least one channel 30, it is preferable that the night-time inserts 25 and the day-time inserts 26 both comprise symmetrical channel(s) 30. The symmetry can be in relation to the transverse center line TCL and/or the longitudinal center line LCL. Preferably for both inserts 25,26, the channel(s) 30 is symmetrical in relation the transverse center line TCL so that the front end and the rear end of the absorbent core will be identical to one other. In other terms, the front end or rear end of the insert 25,26 can be placed in any direction on or within the outer shell 2 and the compartment 15. Additionally, the channel(s) 30 for both inserts 25,26 can be symmetrical in relation to both the transverse center line TCL and the longitudinal center line LCL to further give the impression of identical rear and front ends. The caregiver can thus place the insert 25,26 within the compartment 15 without additional constraints. By comprising a symmetrical channel shape, the care-giver doesn't need to wonder how to correctly position the insert 25,26 within the compartment 15.

According to an embodiment, the first inserts 25 and/or the second inserts 26 comprise at least one distinguishing mean to visually distinguish, or identify, the first inserts 25 from the second insert 26. In embodiments where the first inserts 25 and second inserts 26 have similar visuals aspects, for example if the first inserts 25 comprise synthetic-SAP whereas second inserts 26 comprise bio-SAP, it may beneficial to have a mean to distinguish the two types of inserts so that the care-giver doesn't need to wonder on which inserts 25,26 to secure in the compartment 15. Naturally, the first package comprising a plurality of first inserts 25 can comprise an indicia, or print mark, indicating the nature of the first inserts 25, same as the second package comprising a plurality of second inserts 26 that can also comprise an indicia, or print mark, indicating the nature of the second inserts 26. The distinguishing means comprise for example and not limited to indicia, printing, print marks, writings, drawings, symbols, shapes, logos, channels, a coloured layer such as a coloured topsheet, a coloured backsheet, a coloured core wrap and/or a coloured ADL. For instance if one type of inserts comprise channels and not the other inserts, the presence or absence of channel indicates the nature of the inserts. The same reasoning applies to coloured layers, if one type of inserts have colored topsheets and the other type of inserts have plain white topsheet, the presence or absence of colour indicate the type of the insert. Of course, it is possible to combine several distinguishing means such as the inserts can comprised a coloured topsheet and writings on the topsheet, a coloured backsheet and a channel. The distinguishing means can have other functions such as improving acquisition time and absorbency for the channels, either or both the first and second inserts 25,26 can comprise print marks to indicate how to place the insert 25,26 (up/down, or matching symbols with the outer shell 2) within the compartment 15.

In the embodiments with channels such as illustrated in FIG. 4, preferably, the central section 36 has a length 40 of between 2 and 25% or between 4 and 25%, more preferably between 5 and 20%, even more preferably between 6 and 15 %, most preferably between 7 and 12%, of the absorbent core length L. For example, the ratio between the central section length 40 to the absorbent core length L is greater for the absorbent core 10 of the night-time inserts 25 than for the absorbent core 10 of the day-time inserts 26. If the central section is too short, the absorption time may increase and rewet, to some extent, also may increase. When the central section is too long, it has been found that both absorption time and rewet may greatly increase.

According to an embodiment, the length of the channel CL taken along the longitudinal center line LCL is between 50 and 90%, preferably between 55 and 80%, more preferably between 60 and 75%, of the absorbent core length L for both insert 25,26.

In alternative embodiments, the channel(s) 30 may comprise at least two central sections, six lateral sections, eight diagonal sections and two closing sections. Such pattern may provide a powerful draining system and/or comfort to the wearer.

In accordance with the invention, the channel sections may be straight lines or may be curved lines. Preferably, the closing section is substantially curvilinear in shape, for example in the form of a U-bend with its convex side facing the absorbent core transverse edge at closest proximity, or is substantially linear in shape, for example forming a straight or triangular shape between the lateral sections end points.

According to the invention, the one or more channels 30 are formed by the adhesion of the top layer 35a of the core wrap 35 with the bottom layer 35b of said core wrap 35, in zones substantially free of absorbent material. Such adhesion may advantageously provide core stability and for example reduce the risk of sagging. This adhesion may be provided by any means known in the art to bond core wrap layers together, for example with adhesive bonding or mechanical bonding.

In an embodiment, the top and bottom core wrap layers 35a,35b are adhered by one or more adhesives, *i.e.* adhesive bonding. The adhesive may be either uniformly applied within the channels or it may be applied in zones of the channels such to form zones, preferably alternating zones, of different bonding strength between the core wrap layers. Ways to achieve stronger bonding strength in some zones may include using higher amounts of adhesive in said zones, applying greater mechanical pressure on said zones, or utilizing a different adhesive type.

In an embodiment, the top and bottom core wrap layers 35a,35b are adhered along the channels 30, at a plurality of discrete joining areas, preferably wherein said discrete joining areas are free of adhesive and typically comprise mechanical bonds. Advantageously, this allows for a permanent bonding of the top and bottom layers of the core wrap 35a,35b that limits the presence of additional hydrophobic substances such as adhesives therebetween whilst maximizing the unbonded spaces therebetween to enhance the fluid flow through the channel.

In an embodiment the bonding strength in some zones of the channels is less than in others, and the top core wrap layer and bottom core wrap layer may separate in said zones. This arrangement may allow the bonding in some zones to fail, or temporary bonding, upon for example swelling of the SAP such to allow more volume to be available for expansion thereof (and prevent early saturation or non-optimal absorption), with typically the zones resisting such expansion providing integrity of the channels even in wet state.

According to an embodiment, the first inserts 25 comprise at least one channel 30 comprising a permanent bonding whereas the second inserts 26 comprise at least one channel 30 comprising a temporary bonding.

Of course it is possible to combine several embodiments, for example the array of disposable inserts may comprise first inserts 25 comprising one channel 30 having a greater amount of absorbent material 23 and an ADL 28 with a greater basis weight than the second inserts 26 that comprises one or more channels. Also, the array of disposable inserts may comprise first inserts 25 that don't comprise any channel and comprising synthetic-SAP and an ADL 28 with a greater basis weight than the second inserts 26 comprising bio-SAP and at least one channel 30.

According to the invention, the distribution of synthetic-SAP or bio-SAP can be different between the first inserts 25 and the second inserts 26. Namely, during the day the wearer will be mostly standing up whereas during the night the wearer will be mostly lying down. Hence, according to an embodiment, the distribution of SAP in the first inserts 25 is in majority in the crotch area whereas the distribution of SAP in the second inserts 26 is in majority in the front F and/or back B waist region 3.

In an embodiment, in addition to the channel having the sections as described above, the core further comprises one or more disconnected channels, an advantage being effective added local uniform fluid distribution.

In an embodiment, the width of the channels is constant throughout a continuous path of a channel. Alternatively, it may vary along a channel. The width of the channels may be between 3 and 20 mm, preferably between 5 and 15 mm, more preferably between 5 and 12 mm.

In an embodiment, the absorbent core 10 is a multi-layer core comprising at least a first and a second distinct core layers positioned one on top of the other. The first and/or the second core layer may comprise at least one channel 30. Preferably they both comprise at least one channel 30, wherein the shape and/or dimensions and/or positioning of said channel 30 is substantially identical in both core layers. According to this embodiment, the array comprises a first package comprising a plurality of first inserts 25 comprising a first and a second distinct core layers and a second package comprising a plurality of second inserts 26 comprising solely one core layer. The second core layer positioned under the first core layer may have greater dimensions, meaning length, width and/or height, than the first core layer.

Advantageously, a first core layer comprises a first concentration of superabsorbent polymers therein and a second core layer comprises a second concentration of superabsorbent polymers therein. Said first and second concentrations may be different. The multi-layered core arrangement with differential superabsorbent polymer concentration enables to absorb fluids more efficiently in a multi-fold way and reduce the risk of gel-blocking. The second concentration of superabsorbent polymers, in the bottom core layer, may be greater than the first concentration of superabsorbent polymers, in the top core layer. Preferably said second concentration is at least 1.5, preferably 2, times greater than the first concentration. Such brings advantages such as reduced risk of gel-blocking. The type of superabsorbent polymers may be selected to ensure that the top core layer includes SAP performing well under pressure, to provide a better rewet, and that the bottom core layer includes SAP having fast absorption characteristics. The top core layer may include cellulose fibers and superabsorbent polymers in a proportion of 20-40 Wt% cellulose and 60-80 Wt% SAP; the bottom core layer may include cellulose fibers and superabsorbent polymers in a proportion of 15-30 Wt% cellulose and 70-85 Wt% SAP.

Of course it is possible to combine several embodiments, for example the array of disposable inserts may comprise first inserts 25 comprising one channel 30, a first and a second distinct core layers and having a greater amount of absorbent material 23 and an ADL 28 with a greater basis weight than the second inserts 26 that comprises one or more channels and solely one core layer.

In an embodiment, the acquisition distribution layer 28 is the top layer of the core wrap 35 and the absorbent assembly is free of additional layers, such as an acquisition distribution layer, so that the top layer 35a of the core wrap 35 is in direct contact with the topsheet 8. Said top layer 35a of the core wrap 35 may comprise a spunbond and/or carded, e.g. carded thermobonded, nonwoven layer comprising synthetic fibers, wherein preferably said synthetic fibers are comprised at a level of greater than 80%wt by weight of said layer, and/or wherein said top layer of the core wrap has a basis weight of from 5 to 50 g/m$^2$. Alternatively, said top layer of the core wrap may be multi-layered and comprise at least one spunbond layer and at least one meltblown layer typically the layers being nonwoven layers. Preferably, the multi-layered top layer of the core wrap acting as acquisition distribution system is a nonwoven selected from the group consisting of: SM, SMS, SMMS, and combinations thereof. Advantageously, by choosing core wraps as described, improved performance on rewet may be achieved even whilst eliminating the presence of further acquisition distribution layers, thus further introducing cost benefits.

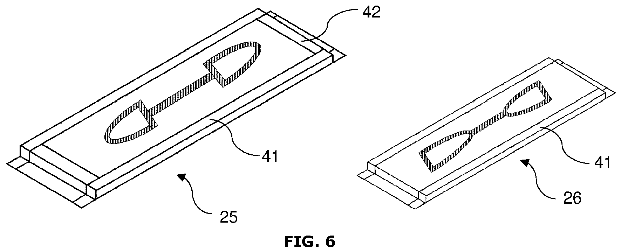

In order to improve absorbency, the night-time insert 25 can have a greater volume than the day-time insert 26. With more volume, the absorbent core 10 is also of greater dimension and thus can absorb more liquid. Hence the night-time inserts 25 have greater total surface area than the second inserts 26 and/or the night-time inserts 25 have greater height than the second inserts 26 as illustrated in FIG. 6. Preferably, the night-time inserts 25 have a greater height than the day-time inserts 26. The outer shell 2 comprising an elastic flexible fastening system and thus can adapt to secure both day-time inserts 26 or night-time inserts 25. Indeed the compartment 15, or pocket, defined by the outer shell 2 (chassis), fastening cuff 14 and elastic mean 16 can adapt to different sizes of inserts 25,26 and maintain said insert 7 within the compartment 15 thanks to the elastic mean 16. The fastening cuff 14 is preferably made out of a resilient, or flexible, material such as polymeric material such as polypropylene, polyethylene, polyester, rayon, nylon, foams, plastic films, formed films, and elastic foams or a combination thereof can be easily deformed. Preferably, given that the outer shell 2 is destined to be washed and re-used, it is preferable to have a washable material such as a 100% polyester, often used in the textile industry, with a thermoplastic polyurethane coating. The outer sell 2 can be made out of cotton, hemp, polyester. The combination of the fastening cuff 14 and the elastic mean 16 also acts as a containment barrier to retain the exudates within the compartment 15 inner volume. The fastening cuff 14 comprises hydrophobic material such as a thermoplastic polyurethane coating to render the fastening cuff 14 more hydrophobic.

The fastening cuff 14 and elastic mean 16 protruding from the outer shell 2 may not be so comfortable for the wearer. According to an embodiment, the insert 7 comprises longitudinal containment flaps 41, or leg cuffs 41, that are adapted to fit about the legs of a wearer when in use and serve as a mechanical barrier to the lateral flow of body exudates until such time as the liquid waste can be absorbed by the absorbent core 10. Such longitudinal containment flaps 41 generally comprise a proximal edge, intended to be attached to the insert 7, for example via adhesive bonding or embossment bonding, and an opposite distal edge which is generally not attached to the insert 7 along at least a portion of its length. An elastic member is generally located adjacent the distal edge to assist in maintaining the containment flap 41 in an upright condition and in maintaining a sealing relationship between the distal edge of the longitudinal containment flap 41 and the body of a wearer during use. The longitudinal containment flaps 41 may be manufactured from a wide variety of materials such as polypropylene, polyester, rayon, nylon, foams, plastic films, formed films, and elastic foams. A number of manufacturing techniques may be used to manufacture the containment flaps 41. For example, the containment flaps may be woven, non-woven, spunbonded, carded, cast, blown or the like. Exemplary of elastic member materials are strands or ribbons of a polymeric, elastomeric material which are adhered to the hybrid diaper at the longitudinal containment flap 41 while in a stretched position, or which are attached to the hybrid diaper while the hybrid diaper is pleated, such that elastic constrictive forces are imparted to the elastic member. Examples of suitable elastomer materials that can be used include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like. The longitudinal containment flaps 41 are preferably made out of a softer material such as meltblown non-woven so that when the longitudinal containment flaps 41 are folded (pivot f - FIG.3) over the fastening cuff 14 and the elastic mean 16, the wearer is in contact with a softer material hence improving comfort.

As illustrated in FIG. 6, the longitudinal containment flaps 41 extend longitudinally along the entire length of the first and second insert 25,26. According to an embodiment, it is preferable to add additional transversal containment flaps 42 that extend transversally along the entire width of the first insert 25. In other terms, the first inserts 25 a greater number of containment flaps 42, or cuffs, than the second inserts 26. Of course, according to an embodiment, the second inserts 26 do not comprise any containment flaps whereas the first inserts 25 comprise longitudinal 41 and/or transversal 42 containment flaps. Again, given that diapers are changed less often during the night, it is preferable to have additional barriers to improve the absorption and improve comfort.

Although absorbency is an important parameter to distinguish night-time inserts 25 from day-time inserts 26, there are other factors that can be taken into consideration. During the day, a toddler will be more active and move restlessly compared to night-time, hence improving the comfort can be more beneficial for day-time inserts 26.

According to an embodiment, the second inserts 26 have anatomical shaped absorbent cores 10, meaning hourglass-shaped (FIG. 4) or T-shaped (FIG. 5) whereas the first inserts 25 have rectangular shaped absorbent cores 10. The cores 10 herein may have various shapes. Preferably the width of the core in the region of the transverse center line is less than in at least one of the front or back portions. This region may be positioned in a crotch portion of the absorbent assembly such to provide better ergonomics and fit along the leg of a wearer especially during the day when the wearer will move more. It is preferred that said cores are symmetric at least about the longitudinal axis thereof. Irrespective of the core geometry, it is understood herein that the same or similar channels as described herein may be interchangeably used.

According to another embodiment, the first inserts 25 have first additives such as skin care additives such as Zinc Oxide, Aloe Vera or a rash preventing cream to treat dermatitis that tends to develop more during the nights because the diapers are less changed, whereas the second inserts 26 have second additives such as odor control additives such as fragrances or perfumes, the first and second additives being different.

As seen previously, diapers are more changed during the day than during the night. Hence it may be preferable that the first inserts 25 comprise a wetness sensor and/or the second inserts 26 comprise wetness indicators. The wetness sensor comprises an electronic system configured to measure data related to a wearer of the absorbent article, and/or to the absorbent article and/or to ambient conditions in close proximity to the wearer of the absorbent article. The electronic system preferably comprises at least one type of sensor and a transmitter, for transmitting measured data related to the absorbent article and/or to ambient conditions in close proximity to a wearer. For example the at least one type of sensor is a humidity sensor and/or a conductance sensor and/or a sensor for measuring ambient parameters and/or a sensor for measuring body parameters and/or a sensor for measuring parameters of the absorbent article and/or any combination thereof. The second inserts 26 may comprise a wetness indicator that is viewable from a garment-facing side of the backsheet. For example, the wetness indicator comprises a composition that changes appearance and/or colour when contacted with urine, the composition comprising a pH indicator and/or a water soluble dye. In this case, at least a portion of the crotch region of the outer shell 2 corresponding with the wetness indicator of the insert is translucent and/or transparent such that said wetness indicator is viewable from a garment facing side of said re-usable outer shell 2 or comprises an optical sensor adapted to automatically detect a colour change of said wetness indicator and send a corresponding signal to an application device.

According to another embodiment, the first inserts 25 comprise a backsheet 9 that is more robust and more breathable than the backsheet comprised in the second inserts 26.

The invention also pertains to an absorbent assembly 1 comprising a re-usable outer shell 2 comprising a chassis and a fastening system 14,16 configured to receive and secure an insert 25,26 within a compartment 15 as described above. The absorbent assembly 1 comprises either a first or second insert 25,26 configured, or dimensioned, to fit within said compartment 15. As stated previously, the outer shell 2 has chassis with a front F and back B waist region 3 having uppermost and lowermost edges, and a crotch region 4 interposed between the front F and back B waist region 3. As described previously, the outer shell 2 comprises a flexible fastening cuff 14 that can be deformed to received inserts 25,26 of different sizes within the compartment. In an embodiment where the night-time insert 25 is thicker, longer or wider than the day-time insert 26, the combination of the fastening cuff 14 that can be deformed and the elastic mean 16 providing elastic strength ensures that the insert 25,26 stays secured within the compartment 15. Dimensions such as thickness, length or width can be measured using any standard method such as with a caliper gauge.

The invention also pertains to an array of absorbent assembly 1 as described above, comprising a first outer shell 2 comprising a chassis and a fastening system 14,16 configured to receive and secure an insert 25,26 within a compartment 15; a second outer shell 2 comprising a chassis and a fastening system 14,16 configured to receive and secure an insert 25,26 within a compartment 15; the first outer shell comprising a bigger length and/or width than the second outer shell, wherein the first or second insert 25,26 are adapted to fit within the compartment 15 of the first and second outer shell 2.

Hence for a care-giver having to take care of two persons of different sizes, it may be more practical for that care-giver to having to buy only one package of inserts that can be fitted onto either outer shell 2 instead of having to buy two packages of inserts each package of inserts being adapted for one type of outer shell 2. Especially when considering day-time inserts 26 and night-times inserts 25, that would represent buying four different packages of inserts (two packages of inserts for each outer shell 2) instead of just two (night-time and day-time) regardless of the size of the outer shell 2, hence an array of disposable inserts also have a use for an array of absorbent assemblies 1. This is possible given the fastening system 14,16 described above.

Absorbency - Method ISO 17190-6:2001

Method ISO 17190-6:2011 comprise the measurement of the centrifuge retention capacity (CRC) of an insert. The "CRC" tests known in the art are designed to measure the amount of saline solution retained inside an insert or inside superabsorbent polymers when under a specific centrifuge force. CRC as referred to herein may be measured by any suitable method known in the art, for example, by following the method described hereinafter: an insert or a sample of said insert (approximately 0.200 grams) placed into a sealable tea bag (7.5 cm * 6.5 cm) and the tea bag sealed. The insert or tea bag are immersed in a 0.9% saline solution for 30 minutes and then centrifuged for three minutes at 1600 rpm on a 21.6 cm diameter centrifuge. The weight difference before centrifuging (dry) and after (wet) is the amount of saline solution absorbed by the insert and this value is the absorption capacity of the insert expressed in g. It is also possible to take this weight difference and divide it by the original dry insert weight and this value is the CRC of the insert expressed in g/g.

This method can also be used to determine the performance of the SAP in terms of absorbency, following the method described hereinafter: approximately 0.200 grams of superabsorbent polymer (synthetic or organic) are placed into a sealable tea bag (7.5 cm * 6.5 cm) and the tea bag sealed. The tea bag and polymer are immersed in a 0.9% saline solution for 30 minutes and then centrifuged for three minutes at 1600 rpm on a 21.6 cm diameter centrifuge. The weight difference before centrifuging and after is the amount of saline solution absorbed by the polymer gel and this value is the absorption capacity of polymer gel expressed in g. It is also possible to take this weight difference and divide it by the original dry polymer gel weight and this value is the CRC of the polymer gel expressed in g/g.

Absorbency - Method ISO 11948-1:1996

Method ISO11948-1:1996 is another method to measure absorbency comprising at least the following steps:

1. Using a balance, measure the dry mass of the insert;
2. Lay said insert on a drainage screen (grid) and lower the drainage screen and insert in a reservoir filled with a 0.9% saline solution and let it soak for 30 minutes;
3. Raise the drainage screen and insert clear of the reservoir and allow excess liquid to drain back under gravity into the reservoir from the urine-absorbing aid for 5 min.

4. Using the balance, measure the wet mass of insert.
5. Subtract the dry mass measure in step 1 of the insert from the wet mass measured in step 4 to give the absorption capacity in grams.

AUL (Absorbency Under Load, 0.7 psi) - Test Method

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as W0. Then 0.900 +/- 0.005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

$$AUL0.7psig/g=Wb-Wa/Wa-W0$$

AUL 0.3 psi and 0.5 psi are measured similarly at the appropriate lower pressure.

**Claims**

1. Array of disposable inserts (25,26) for an absorbent assembly (1), the array comprising:

   a. a first package comprising a plurality of first inserts (25) for an absorbent assembly (1), configured to be arranged on or at least partially within an outer shell (2);
   b. a second package comprising a plurality of second inserts (26) for absorbent assembly (1), configured to be arranged on or at least partially within an outer shell (2);
   c. each first and second insert (25,26) comprising a topsheet (8), a backsheet (9) and an absorbent core (10) comprising absorbent material (23) arranged in between the topsheet (8) and backsheet (9);

   the first insert (25) comprising a first absorbency as measured by method mentioned in the description and the second insert (26) comprising a second absorbency as measured by method mentioned in the description;
   wherein, the first absorbency is greater than the second absorbency.

2. Array of disposable inserts (25,26) according to claim 1, wherein the absorbent material (23) comprises superabsorbent polymers (22) comprising synthetic hydrogel-forming polymers (SAP) and/or superabsorbent polymers (22) comprising natural hydrogel-forming polymers (bio-SAP).

3. Array of disposable inserts (25,26) according to claim 1 or 2, wherein the first inserts (25) comprise a greater amount of absorbent material (23) than the second inserts (26), preferably the first inserts (25) comprise a greater amount of superabsorbent polymers (22) comprising synthetic hydrogel-forming polymers (SAP) and/or superabsorbent polymers (22) comprising natural hydrogel-forming polymers (bio-SAP).

4. Array of disposable inserts (25,26) according to claim 2 or 3, wherein the first inserts (25) comprise superabsorbent polymers (22) comprising synthetic hydrogel-forming polymers (SAP) whereas the second inserts (26) comprise superabsorbent polymers (22) comprising natural hydrogel-forming polymers (bio-SAP).

5. Array of disposable inserts (25,26) according to any of the preceding claims, wherein the first inserts (25) comprise first superabsorbent polymer grades (SAP1) that has an Absorption Under Load greater than 15 g/g as measured by method mentioned in the description, whereas the second inserts (26) comprise second superabsorbent polymer grades (SAP2) that has an Absorption Under Load lesser than 15 g/g as measured by method mentioned in the description.

6. Array of disposable inserts (25,26) according to any of the preceding claims, wherein the first inserts (25) comprise a first acquisition distribution layer (28) arranged between the absorbent core (10) and the topsheet (8) with a first basis weight and height whereas the second inserts (26) comprise a second acquisition distribution layer (28) arranged between the absorbent core (10) and the topsheet (8), said second acquisition distribution layer (28) having a basis weight or a height that is lesser than the basis weight or height of the first acquisition distribution layer (28) or whereas the second inserts (26) comprise an absorbent core (10) that is directly bonded to the topsheet (8).

7. Array of disposable inserts (25,26) according to any of the preceding claims, wherein the first inserts (26) comprise an absorbent core (10) comprising at least one channel (30) substantially free of absorbent material (23) whereas the second inserts (26) comprise channels (30) substantially free of absorbent material (23) covering a greater surface area.

8. Array of disposable inserts (25,26) according to claim 7, wherein the first inserts (25) comprise at least one channel (30) substantially free of absorbent material (23) comprising a permanent bonding whereas the second inserts (26) comprise at least one channel (30) substantially free of absorbent material (23) comprising a temporary bonding.

9. Array of disposable inserts (25,26) according to any of the preceding claims, wherein the absorbent material (23) comprises cellulosic fibers (21) wherein the first inserts (25) comprise a first amount of cellulosic fibers (21) and the second inserts (26) comprise a second amount of cellulosic fibers (21), the first amount of cellulosic fibers (21) being greater than the second amount of cellulosic fibers (21).

10. Array of disposable inserts (25,26) according to any of the preceding claims, wherein the first inserts (25) have a first volume and the second inserts (26) have a second volume, the first volume being greater than the second volume.

11. Array of disposable inserts (25,26) according to any of the preceding claims, wherein the first inserts (25) have first additives such as skin care additives and/or the second inserts (26) have second additives such as odor control additives, the first additives being different than the second additives.

12. Array of disposable inserts (25,26) according to any of the preceding claims, wherein the first inserts (25) comprise longitudinal containment flaps (41) and preferably transversal containment flaps (42), wherein the second inserts (26) have first cuffs longitudinal containment flaps (41).

13. Array of disposable inserts (25,26) according to any of the preceding claims, wherein the first inserts (25) comprise wetness sensors and/or the second inserts (26) comprise wetness indicators.

14. Absorbent assembly (1) comprising:

   a. a re-usable outer shell (2) comprising a chassis and a fastening system (14,16) configured to receive and secure an insert (25,26) at least partially within a compartment (15);
   b. a first or second insert (25,26) as described in any of the claims 1 to 13 configured to fit at least partially within said compartment (15).

15. Absorbent assembly (1) according to claim 13, wherein the outer shell (2) comprises a flexible cuff (14) that can be deformed to received inserts of different sizes at least partially within the compartment (15).

16. Array of absorbent assemblies according to claim 14 or 15, comprising

   a. a first outer shell (2) comprising a chassis and a fastening system (14,16) configured to receive and secure an insert (25,26) at least partially within a compartment (15);
   b. a second outer shell (2) comprising a chassis and a fastening system (14,16) configured to receive and secure an insert (25,26) at least partially within a compartment (15);
   c. the first outer shell comprising a bigger size than the second outer shell;
   d. wherein the first or second insert (25,26) are configured to fit at least partially within the compartment (15) of the first and second outer shell (2).

**FIG. 1**

**FIG. 2**

**FIG. 3**

**FIG. 4**

**FIG. 5**

**FIG. 6**

| | Europäisches Patentamt European Patent Office Office européen des brevets | EUROPEAN SEARCH REPORT | Application Number EP 21 21 4838 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2014 039869 A (PROCTER & GAMBLE) 6 March 2014 (2014-03-06) | 1-16 | INV. A61F13/505 |
| Y | * paragraphs [0001], [0027], [0103], [0167], [0168], [0169], [0174] - [0176]; figures 1-4 * | 7 | A61F13/70 A61F13/78 A61F13/80 |
| X | EP 3 842 017 A1 (ONTEX BV [BE]; ONTEX GROUP NV [BE]) 30 June 2021 (2021-06-30) * paragraphs [0001], [0006] - [0009], [0041], [0042], [0048] - [0063]; figures 1,2 * | 14,15 | |
| Y | EP 3 900 686 A1 (ONTEX BV [BE]; ONTEX GROUP NV [BE]) 27 October 2021 (2021-10-27) * claims 1-18 * | 7 | |
| A | US 2021/000661 A1 (GUIDOTTI EDWARD [SE] ET AL) 7 January 2021 (2021-01-07) * claims 1-19; figures 1-3 * | 1-16 | |
| A | US 2014/005622 A1 (WIRTZ BIRGIT [DE] ET AL) 2 January 2014 (2014-01-02) * claims 1-20 * | 1-16 | TECHNICAL FIELDS SEARCHED (IPC) A61F |
| A | US 2013/211355 A1 (NISHIKAWA MASAHARU [JP] ET AL) 15 August 2013 (2013-08-15) * claims 1-20 * | 1-16 | |
| A | US 2016/100999 A1 (HAMILTON RAYMOND SCOTT [US] ET AL) 14 April 2016 (2016-04-14) * claims 21,22 * | 1-16 | |
| A | US 2016/374871 A1 (SEITZ BRET DARREN [US] ET AL) 29 December 2016 (2016-12-29) * claims 1-20 * | 1-16 | |

The present search report has been drawn up for all claims

| Place of search | Date of completion of the search | Examiner |
|---|---|---|
| Munich | 8 June 2022 | Beins, Ulrika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT
## ON EUROPEAN PATENT APPLICATION NO.

**EP 21 21 4838**

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2022

| Patent document cited in search report | Publication date | Patent family member(s) | | Publication date |
|---|---|---|---|---|
| JP 2014039869 A | 06-03-2014 | AR | 075333 A1 | 23-03-2011 |
| | | AU | 2010204685 A1 | 28-07-2011 |
| | | AU | 2010204749 A1 | 28-07-2011 |
| | | BR | PI1006800 A2 | 12-04-2016 |
| | | BR | PI1006802 A2 | 12-04-2016 |
| | | BR | PI1006884 A2 | 15-03-2016 |
| | | BR | PI1007492 A2 | 16-02-2016 |
| | | CA | 2749604 A1 | 22-07-2010 |
| | | CA | 2749609 A1 | 22-07-2010 |
| | | CA | 2749626 A1 | 22-07-2010 |
| | | CA | 2749747 A1 | 22-07-2010 |
| | | CN | 102281843 A | 14-12-2011 |
| | | CN | 102281845 A | 14-12-2011 |
| | | CN | 102281848 A | 14-12-2011 |
| | | CN | 102281850 A | 14-12-2011 |
| | | EP | 2376039 A1 | 19-10-2011 |
| | | EP | 2376040 A1 | 19-10-2011 |
| | | EP | 2376041 A1 | 19-10-2011 |
| | | EP | 2376046 A1 | 19-10-2011 |
| | | ES | 2593081 T3 | 05-12-2016 |
| | | JP | 5497069 B2 | 21-05-2014 |
| | | JP | 5808785 B2 | 10-11-2015 |
| | | JP | 2012515056 A | 05-07-2012 |
| | | JP | 2012515057 A | 05-07-2012 |
| | | JP | 2012515058 A | 05-07-2012 |
| | | JP | 2012515059 A | 05-07-2012 |
| | | JP | 2014039869 A | 06-03-2014 |
| | | PL | 2376046 T3 | 30-12-2016 |
| | | RU | 2011121645 A | 10-12-2012 |
| | | RU | 2011124945 A | 27-12-2012 |
| | | SG | 172966 A1 | 29-08-2011 |
| | | SG | 172967 A1 | 29-08-2011 |
| | | US | 2010179496 A1 | 15-07-2010 |
| | | US | 2010179500 A1 | 15-07-2010 |
| | | US | 2010179501 A1 | 15-07-2010 |
| | | US | 2011270211 A1 | 03-11-2011 |
| | | WO | 2010083260 A1 | 22-07-2010 |
| | | WO | 2010083287 A1 | 22-07-2010 |
| | | WO | 2010083290 A1 | 22-07-2010 |
| | | WO | 2010083293 A1 | 22-07-2010 |
| | | ZA | 201104165 B | 23-12-2015 |
| EP 3842017 A1 | 30-06-2021 | EP | 3842017 A1 | 30-06-2021 |
| | | WO | 2021130165 A1 | 01-07-2021 |
| EP 3900686 A1 | 27-10-2021 | NONE | | |

EPO FORM P0459

For more details about this annex : see Official Journal of the European Patent Office, No. 12/82

**page 1 of 2**

## ANNEX TO THE EUROPEAN SEARCH REPORT
## ON EUROPEAN PATENT APPLICATION NO.

**EP 21 21 4838**

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2022

| Patent document cited in search report | | Publication date | Patent family member(s) | | Publication date |
|---|---|---|---|---|---|
| US 2021000661 | A1 | 07-01-2021 | BR | 112020017496 A2 | 22-12-2020 |
| | | | CN | 111801081 A | 20-10-2020 |
| | | | EP | 3773395 A1 | 17-02-2021 |
| | | | JP | 2021518238 A | 02-08-2021 |
| | | | US | 2021000661 A1 | 07-01-2021 |
| | | | WO | 2019185111 A1 | 03-10-2019 |
| US 2014005622 | A1 | 02-01-2014 | EP | 2679209 A1 | 01-01-2014 |
| | | | JP | 6087428 B2 | 01-03-2017 |
| | | | JP | 2015519185 A | 09-07-2015 |
| | | | US | 2014005622 A1 | 02-01-2014 |
| | | | WO | 2014004283 A1 | 03-01-2014 |
| US 2013211355 | A1 | 15-08-2013 | CA | 2864516 A1 | 22-08-2013 |
| | | | CN | 104244888 A | 24-12-2014 |
| | | | EP | 2814440 A1 | 24-12-2014 |
| | | | JP | 2015506788 A | 05-03-2015 |
| | | | US | 2013211355 A1 | 15-08-2013 |
| | | | WO | 2013122936 A1 | 22-08-2013 |
| US 2016100999 | A1 | 14-04-2016 | CN | 107072842 A | 18-08-2017 |
| | | | CN | 111544205 A | 18-08-2020 |
| | | | EP | 3203959 A1 | 16-08-2017 |
| | | | EP | 3659564 A1 | 03-06-2020 |
| | | | US | 2016100999 A1 | 14-04-2016 |
| | | | US | 2020330289 A1 | 22-10-2020 |
| | | | WO | 2016057736 A1 | 14-04-2016 |
| US 2016374871 | A1 | 29-12-2016 | CN | 107787212 A | 09-03-2018 |
| | | | EP | 3313344 A1 | 02-05-2018 |
| | | | EP | 3957290 A1 | 23-02-2022 |
| | | | JP | 6633106 B2 | 22-01-2020 |
| | | | JP | 2018522645 A | 16-08-2018 |
| | | | US | 2016374871 A1 | 29-12-2016 |
| | | | US | 2019388283 A1 | 26-12-2019 |
| | | | WO | 2016209719 A1 | 29-12-2016 |

EPO FORM P0459

For more details about this annex : see Official Journal of the European Patent Office, No. 12/82

**EP 4 197 508 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7431716 B